Europäisches Patentamt

(19) European Patent Office

Office européen des brevets

(11) **EP 0 669 125 B1**

## (12) FASCICULE DE BREVET EUROPEEN

(45) Date de publication et mention
de la délivrance du brevet:
**24.01.2001 Bulletin 2001/04**

(51) Int Cl.⁷: **A61K 7/42**

(21) Numéro de dépôt: **95400173.1**

(22) Date de dépôt: **26.01.1995**

(54) **Compositions cosmétiques autobronzantes à base de dihydroxyacétone**

Selbstbräunende kosmetische Mittel enthaltend Dihydroxyaceton

Self tanning cosmetic compositions containing dihydroxyacetone

(84) Etats contractants désignés:
**DE ES FR GB IT**

(30) Priorité: **28.02.1994 FR 9402254**

(43) Date de publication de la demande:
**30.08.1995 Bulletin 1995/35**

(73) Titulaire: **L'OREAL**
**75008 Paris (FR)**

(72) Inventeurs:
• **Ascione, Jean-Marc**
  **F-75018 Paris (FR)**
• **Allard, Delphine**
  **F-92700 Colombes (FR)**

• **Hansenne, Isabelle**
  **F-75017 Paris (FR)**

(74) Mandataire: **Andral, Christophe André Louis**
  **L'OREAL-DPI**
  **6 rue Bertrand Sincholle**
  **92585 Clichy Cedex (FR)**

(56) Documents cités:
**WO-A-93/16683**          **FR-A- 2 597 345**

Remarques:
Le dossier contient des informations techniques
présentées postérieurement au dépôt de la
demande et ne figurant pas dans le présent
fascicule.

## Description

La présente invention concerne de nouvelles compositions cosmétiques à usage topique plus particulièrement destinées au bronzage et/ou au brunissage artificiel de la peau (compositions ci-après dénommées plus simplement compositions autobronzantes), un de leur procédé de préparation, ainsi que leur utilisation dans l'application cosmétique susmentionnée. Plus précisément encore, elle concerne des compositions autobronzantes à activité améliorée se présentant sous la forme d'émulsions de type huile-dans-eau particulières (support cosmétiquement acceptable) et comprenant, à titre d'agent d'autobronzage, de la dihydroxyacétone.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres). Pour diverses raisons liées en particulier à un meilleur confort d'utilisation (douceur, émollience, facilité d'application), les compositions autobronzantes actuelles se présentent le plus souvent sous la forme d'une émulsion de type huile-dans-eau (c'est à dire un support constitué d'une phase continue dispersante aqueuse et d'une phase discontinue dispersée huileuse) dans laquelle on a introduit, à des concentrations diverses, de la dihydroxyacétone qui, compte tenu de son caractère hydrophile, se retrouve présente dans la phase aqueuse de l'émulsion. De telles émulsions sont décrites dans la demande internationale WO93/16683. Elles sont préparées selon un procédé dans lequel la phase aqueuse et la phase huileuse sont chauffées de façon séparée à environ 85°C puis mélangées ; on procède ensuite à une étape d'émulsification par refroidissement du mélange à environ 40-45°C et la dihydroxyacétone est dissoute dans l'eau et introduite dans l'émulsion ainsi formée. Dans ces émulsions classiques, qui contiennent en outre des agents émulsionnants (ou tensio-actifs) et d'éventuels additifs cosmétiques usuels tels que parfums, colorants ou conservateurs, la taille des globules constituant la phase liquide huileuse est généralement supérieure à plusieurs micromètres.

Toutefois, l'un des inconvénients des compositions autobronzantes connues à ce jour et appartenant au type ci-dessus (émulsion H/E contenant de la DHA) est que l'intensité de la coloration obtenue sur la peau et/ou la rapidité avec laquelle cette coloration se développe, peuvent apparaître comme encore insuffisantes.

La présente invention a notamment pour but de résoudre le problème ci-dessus en proposant des émulsions de type H/E à base de DHA qui présentent une efficacité et/ou une activité autobronzantes sur la peau améliorées.

Ainsi, à la suite d'importantes recherches menées sur la question, il a maintenant été trouvé par la Demanderesse, et ceci de façon tout à fait inattendue et surprenante, qu'il est possible d'améliorer le pouvoir de coloration cutanée attaché aux émulsions H/E classiques de l'art antérieur à base de DHA, en mettant en oeuvre des émulsions H/E spécifiques dites "ultrafines", pour lesquelles la taille des globules constituant la phase huileuse est comprise dans des limites bien déterminées, lesdites émulsions ultrafines de type H/E étant elles-mêmes de préférence obtenues selon la technique dite "d'inversion de phase" qui sera détaillée par la suite. Toutes choses étant égales par ailleurs (i.e. à composition chimique et concentrations identiques), on observe qu'une composition autobronzante conforme à l'invention, par le simple ajustement de la taille des globules huileux à une valeur convenable indiquée ci-après, présente systématiquement, au niveau de son pouvoir de coloration de la peau, des propriétés améliorées par rapport à une même composition autobronzante ne satisfaisant pas au critère susmentionné de taille des globules d'huile. Par ailleurs, il a été constaté de manière surprenante que, dans les compositions conformes à l'invention, la DHA présente une stabilité chimique nettement accrue (moindre décomposition au cours du temps).

Ces découvertes sont à la base de la présente invention.

Il convient de noter ici que dans la demande de brevet français FR-A- 2 597 345 au nom de la Demanderesse, il a été suggéré d'introduire de la dihydroxyacétone dans des dispersions aqueuses de sphérules lipidiques mésomorphes multilamélaires (niosomes ou liposomes), lesdites dispersions pouvant en outre contenir, dans la phase aqueuse externe dispersante, une phase huileuse liquide dispersée ; toutefois, et comme le montrera l'un des exemples donné ci-après, même dans le cas où cette phase huileuse complémentaire serait constituée de globules huileux de taille moyenne conforme à la présente invention (voir en particulier l'exemple 4 de la demande précitée), on n'observe pas, pour de telles dispersions, les effets avantageux attachés aux émulsions de la présente invention.

Ainsi conformément à l'un des objets de la présente invention, il est maintenant proposé des compositions cosmétiques à usage topique utilisable pour le bronzage et/ou le brunissage artificiels de la peau, comprenant au moins de la dihydroxyacétone dans un support cosmétiquement acceptable constitué d'une émulsion ultrafine de type huile-dans-eau exempte de vésicules lipidiques, et qui sont caractérisées par le fait que la taille moyenne des globules huileux mesurée au microscope optique est comprise entre 100 nm et 1000 nm.

Le procédé de préparation de l'émulsion ultrafine de type huile-dans-eau, conformément au libellé des revendications 1 à 3, et l'émulsion ultrafine susceptible d'être obtenue selon ce procédé, conformément au libellé des revendications 4 à 11, constituent deux autres objets de la présente invention.

D'autres caractéristiques, aspects et avantages de l'invention apparaitront à la lecture de la description détaillée qui va suivre.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type huile-dans-eau. En particulier, ces composés peuvent être choisis, seuls ou en mélanges, parmi les différents corps gras, les huiles d'origine végétale, animale ou minérale, les cires naturelles ou synthétiques, par ex.

Parmi les huiles pouvant rentrer dans la composition de la phase grasse, on peut notamment citer :

- les huiles minérales telles que l'huile de paraffine et l'huile de vaseline,

- les huiles d'origine animale telles que le perhydrosqualène,

- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,

- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-$\alpha$-oléfines.

Comme autres huiles utilisables dans les émulsions selon l'invention, on peut encore citer les benzoates d'alcools gras en $C_{12}$-$C_{15}$ (Finsolv TN de FINETEX), les alcools gras tels que l'alcool laurique, cétylique, myristique, stéarilique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en $C_{10}$-$C_{18}$, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déja utilisés de manière habituelle dans la fabrication et l'obtention des compositions cosmétiques autobronzantes.

Selon une caractéristique essentielle de la présente invention, la taille moyenne des particules (ou globules) de phase grasse au sein de la phase aqueuse dispersante doit être comprise dans des limites bien particulières, à savoir entre 100 nm et 1000 nm. De préférence, cette taille moyenne est comprise entre 100 nm et 500 nm. Encore plus préférentiellement, la distribution en taille des globules huileux est telle que la plupart desdits globules (i.e au moins 90% en nombre) présentent une taille comprise entre les bornes indiquées ci-avant.

De manière classique, la phase aqueuse dispersante peut être constituée par de l'eau, ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles.

Comme indiqué précédemment, la dihydroxyacétone, compte tenu de son caractère hydrosoluble et hydrophile, est présente dans la phase aqueuse des émulsions selon l'invention.

Parmi les adjuvants cosmétiques classiques susceptibles d'être contenus dans la phase aqueuse et/ou dans la phase grasse des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, ou tout autre ingrédient habituellement utilisé dans le domaine des produits autobronzants.

Les émulsions conformes à l'invention contiennent en outre généralement des tensio-actifs ou émulsionnants particuliers dont l'emploi a été rendu nécessaire pour la préparation et l'obtention de l'émulsion ultrafine. Ce point sera détaillé par la suite. Elles peuvent en outre contenir des co-émulsionnants spécifiques dont le rôle est, lors de la préparation de l'émulsion, de diminuer de manière substantielle la quantité d'agents tensio-actifs nécessaire à la réalisation de l'émulsion.

A titre indicatif, les formulations autobronzantes conformes à l'invention présentent généralement les compositions suivantes :

(i) phase aqueuse : de 50 à 95 % en poids, de préférence de 70 à 90 % en poids, par rapport à l'ensemble de la formulation,

(ii) phase huileuse : de 5 à 50 % en poids, de préférence de 10 à 30 % en poids, par rapport à l'ensemble de la formulation,

(iii) dihydroxyacétone : de 0,5 à 10 % en poids, de préférence de 1 à 7 % en poids, par rapport à l'ensemble de la formulation,

(iv) émulsionnant(s) : de 0,5 à 20 % en poids, de préférence de 2 à 10% en poids, par rapport à l'ensemble de la formulation.

Le procédé préféré de préparation des émulsions ultrafines selon l'invention, lui-même constitutif d'un second objet de la présente invention, va maintenant être développé.

Comme indiqué précédemment, ce procédé repose sur la technique de fabrication des émulsions H/E par inversion de phase. Cette technique est, dans son principe, bien connue de l'homme de l'art et est notamment décrite dans l'article "Phase Inversion Emulsification", par Th Förster et al, paru dans Cosmetics & Toiletries, vol. 106, Décembre 1991, pp 49-52. Son principe est ainsi le suivant : on prépare une émulsion (introduction de l'eau dans l'huile) à une température qui doit être supérieure à la température d'inversion de phase (ou TIP) du système, c'est à dire la température à laquelle l'équilibre entre les propriétés hydrophiles et lipophiles du ou des émulsionnants mis en oeuvre est atteint à température élevée (>TIP), l'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion.

Selon l'invention, de la DHA doit être présente dans l'émulsion ultrafine H/E finale. Ainsi, selon une première variante de mise en oeuvre du procédé de préparation selon l'invention, l'inversion de phase de l'émulsion est conduite en présence de la DHA (cette DHA est de préférence contenue dans la phase aqueuse initiale) selon une deuxième variante, qui est ici préférée, de mise en oeuvre de ce procédé, cette DHA n'est introduite qu'après que l'émulsion par inversion de phase ait été obtenue. Il est bien entendu possible de cumuler les deux variantes.

L'une des difficultés pour la mise en oeuvre d'un procédé tel que ci-dessus réside dans le choix convenable du système émulsionnant qui doit être approprié au résultat recherché.

Les systèmes émulsionnants qui doivent ainsi être retenus dans le cadre de l'invention sont ceux qui permettent effectivement d'obtenir des émulsions ultrafines de type H/E par inversion de phase (100 nm$<\Phi_{globules}<$1000 nm) et dans lesquelles la DHA ne se trouve finalement présente uniquement ou essentiellement que dans la phase aqueuse dispersante.

Les travaux de la Demanderesse ont montré que, à cet effet, les systèmes émulsionnants convenant à la présente invention devaient être des émulsionnants de type non ioniques et, plus particulièrement encore, être choisis parmi les alcools gras polyoxyéthylénés et/ou polyoxypropylénés (i.e des composés obtenus par réaction entre un alcool gras aliphatique, comme l'alcool béhénique ou l'alcool cétylique, avec de l'oxyde d'éthylène ou de l'oxyde de propylène ou un mélange oxyde d'éthylène/oxyde de propylène) et les esters d'acides gras et de polyols, éventuellement polyoxyéthylénés et/ou polyoxypropylénés (i.e des composés obtenus par réaction d'un acide gras, comme l'acide stéarique ou l'acide oléique, avec un polyol, comme par exemple un alkylèneglycol ou du glycérol ou un polyglycérol, éventuellement en présence d'oxyde d'éthylène ou d'oxyde de propylène ou d'un mélange oxyde d'éthylène/oxyde de propylène), ou leurs mélanges. Par ailleurs, et de préférence, le système émulsionnant retenu présentera un HLB global (comme cela est bien connu, on désigne par HLB (Hydrophilic-Lipophilic Balance, au sens de Griffin voir J. Soc. Cosm. Chem. 1954 (vol 5), pp 249-256) l'équilibre entre le caractère hydrophile et le caractère lipophile de l'agent tensioactif) compris entre 9,5 et 11,5, avantageusement proche de 10, de manière à permettre l'obtention d'une inversion de phase à une température inférieure à 90°C (TIP<90°C).

Les détails du procédé de préparation selon l'invention apparaitront dans les exemples donnés ci-après.

Un quatrième objet de la présente invention est constitué par l'utilisation des émulsions ultrafines de type huile-dans-eau selon l'invention telles que ci-dessus définies comme, ou pour la fabrication de, compositions cosmétiques pour le bronzage et/ou le brunissage artificiels de la peau. Les compositions peuvent alors être conditionnées sous la forme de crèmes, de laits, de gels crèmes, ou bien encore de lotions fluides, en particulier de lotions fluides vaporisables (les compositions selon l'invention présentant en effet la propriété avantageuse complémentaire d'être aisément diluables à l'eau).

Le procédé de traitement cosmétique de la peau destiné à la bronzer et/ou la brunir artificiellement consiste à appliquer sur celle-ci une quantité efficace d'une composition cosmétique telle que définie plus haut.

Des exemples concrets, mais nullement limitatifs, illustrant l'invention, vont maintenant être donnés.

## EXEMPLE 1

Dans cet exemple, on a préparé et comparé deux émulsions de même composition chimique à base de dihydroxyacétone, l'une étant conforme à l'invention (E1), ultrafine et obtenue par inversion de phase, l'autre comparative (E2), non ultrafine ($\Phi_{globules}$>1 μm) et obtenue sans mettre en oeuvre d'inversion de phase.

Les compositions chimiques (% en poids ramenés à l'ensemble de la formulation) de ces émulsions étaient les suivantes :

*Phase A* :

- Alcool cétylstéarylique à 15 moles d'oxyde d'éthylène (MERGITAL CS 15 de HENKEL)     3,3 %
- Stéarate de glycérol (TEGIN 90 de GOLDSCHMIDT)     1,7 %
- Di-n-octyléther (CETIOL OE de HENKEL)     9 %
- Dioctylcyclohexane (CETIOL S de HENKEL)     9 %
- Cyclométhicone (DC 245 Fluid de DOW CORNING)     4,5 %

*Phase B* :

- Dihydroxyacétone     5 %
- Eau     30 %

*Phase C* :

- Glycérine     2,5 %
- Eau     qsp     100 %

*Phase D* :

- Parfum     qs
- Conservateurs     qs

Le mode opératoire qui a été suivi pour préparer ces deux émulsions était le suivant : les phases grasse (*A*) et aqueuse (*C*) ont été toutes deux préalablement portées à une température de l'ordre de 90°C ; puis on a ajouté la phase aqueuse (*C*) dans la phase grasse (*A*), et ceci sous agitation énergique de cette dernière au moyen d'une turbine type MORITZ (1000 t/mn) : dans le cas de l'émulsion E1, cette étape d'émulsification a été conduite à 80°C, c'est à dire à une température supérieure à la température d'inversion de phase du système, laquelle est ici de 72°C (TIP), alors que dans le cas de l'émulsion E2, on a conduit l'émulsification à 50°C (i.e. T°C<TIP) ; et enfin, on a introduit dans l'émulsion résultante, vèrs 40 °C, d'abord la phase (*B*) puis la phase (*D*).

On a ensuite comparé les caractéristiques relatives d'une part à la taille des globules d'huile (microscope optique G x 400) et, d'autre part, au pouvoir de coloration sur la peau, des deux émulsions E1 et E2 ainsi obtenues. Le pouvoir de coloration a été apprécié au moyen du test suivant : on a appliqué les émulsions, à raison de 2 mg/cm$^2$ de peau (carrés de 6 cm$^2$ environ), sur les avant-bras de trois personnes témoins (P1, P2 et P3) et on a mesuré sur ces avant-bras l'écart colorimétrique de la valeur L (coordonnée chromatique de luminance, mesurée à l'aide d'un colorimètre MINOLTA CM 1000) avant (To) et après (24 heures) application, de manière à déterminer une valeur absolue ΔL qui rend compte de l'intensité de la coloration obtenue sur la peau après application (plus le ΔL est élevé plus la coloration est intense) :

$$\Delta L = L_{To} - L_{T24h}$$

Les résultats sont rassemblés dans le tableau I ci-dessous. Ces résultats montrent clairement que l'émulsion E1 conforme à l'invention apporte sur la peau, et ceci de façon significative, une coloration plus intense que l'émulsion comparative E2, 24 heures après application.

TABLEAU I

| | | FORME DE L'EMULSION | COLORATION ($\Delta L$) | | | |
|---|---|---|---|---|---|---|
| | | | P1 | P2 | P3 | M[1] ($\sigma$[2]) |
| | E1 | ULTRAFINE et homogène, pas de globules huileux visibles au microscope optique ($\Phi < 1 \ \mu m$) | 6,1 | 4,8 | 5,9 | 5,6 (0,7) |
| | E2 | émulsion grossière, globules huileux visibles au microscope optique ($\Phi > 1 \ \mu m$) | 4,1 | 3,8 | 3,8 | 3,9 (0,2) |

**[1]**: moyenne

**[2]** : écart-type

## EXEMPLE 2

Dans cet exemple, on a comparé l'émulsion E1 conforme à l'invention de l'exemple 1, à une formule niosomée E3 contenant également 5% en poids de DHA et préparée selon le mode opératoire indiqué à l'exemple 4 de la demande FR-A- 2 597 345 (dans ce cas, la DHA est introduite dans la dernière étape du procédé).

La composition chimique (% en poids ramenés à l'ensemble de la formulation) de cette formulation comparative niosômée étaient la suivante:

_Vésicules lipidiques (niosomes) dispersés_ ($\Phi_{vésicules}$ = 0.5 µm environ) :

- Alcool cétylique polyglycérolé à 3 moles de glycérol (CHIMEXANE NL de CHIMEX)    3,8 %
- Cholestérol    3,8 %
- Stéaroyl Glutamate Monosodique    0,4 %

_Phase huileuse liquide dispersée_ ($\Phi_{globules} < 1$ µm) :

- Huile de vaseline    5 %
- Adipate de diisopropyle    12 %

_Phase aqueuse dispersante_ :

- Glycérine    5 %
- Dihydroxyacétone    5 %
- Hydroxyéthylcellulose    0,5 %
- Parfum    qs
- Conservateurs    qs
- Eau    qsp    100 %

En suivant le test donné à l'exemple 1, on a ensuite évalué le pouvoir de coloration sur peau de chacune des deux compositions, 24 heures après application ($T_{24h}$). Les résultats sont donnés dans le tableau II ci-dessous. Ces résultats montrent clairement que l'émulsion E1 conforme à l'invention apporte sur la peau, et ceci de façon significative, une coloration plus intense que l'émulsion comparative E3, 24 heures après application.

TABLEAU II

| | COLORATION ($\Delta$L) | | | |
|---|---|---|---|---|
| | P4 | P5 | P6 | M[1] ($\sigma$[2]) |
| E1 | 6,6 | 5,4 | 7,5 | 6,5 (1,0) |
| E3 | 5,6 | 4,4 | 6,0 | 5,3 (0,8) |

[1] : moyenne

[2] : écart-type

## Revendications

1.  Procédé de préparation d'une émulsion ultrafine de type huile-dans-eau exempte de vésicules lipidiques comprenant au moins de la dihydroxyacétone et dont la taille moyenne des globules qui constituent la phase huileuse de ladite émulsion, mesurée au microscope optique est comprise entre 100 et 1000 nm, caractérisé par le fait qu'il comprend les étapes suivantes :

    (i) on mélange, en présence d'un système émulsionnant convenable et sous agitation, une phase grasse d'une part et une phase aqueuse d'autre part, ledit mélange se faisant à une température supérieure à la température d'inversion de phase (TIP) du milieu, de manière à obtenir une émulsion de type eau-dans-huile,
    (ii) on ramène la température de l'émulsion ainsi obtenue à une température inférieure à ladite température d'inversion de phase, ce par quoi l'on obtient une émulsion ultrafine de type huile-dans-eau,
    (iii) on procède à l'introduction de dihydroxyacétone lors de la mise en oeuvre de l'étape (i) et/ou à l'issue de l'étape (ii), mais de préférence à l'issue de l'étape (ii).

2.  Procédé selon la revendication 1, caractérisé en ce que le ou les agents émulsionnants sont de type non ioniques et sont choisis, seuls ou en mélanges, parmi les alcools gras polyoxyéthylénés et/ou polyoxypropylénés, et les esters d'acides gras et de polyols, éventuellement polyoxyéthylénés et/ou polyoxypropylénés.

3.  Procédé selon la revendication 2, caractérisé en ce que le système émulsionnant présente un HLB global compris entre 9,5 et 11,5, et de préférence proche de 10.

4.  Emulsion ultrafine de type huile-dans-eau exempte de vésicules lipidiques, comprenant au moins de la dihydroxyacétone et dont la taille moyenne des globules huileux, mesurée au microscope optique est comprise entre 100 nm et 1000 nm, susceptible d'être obtenue selon le procédé défini dans l'une quelconque des revendications 1 à 3.

5.  Emulsion selon la revendication 4, caractérisée en ce que ladite taille moyenne est comprise entre 100 et 500 nm.

6.  Emulsion selon l'une quelconque des revendications 4 ou 5, caractérisée en ce qu'au moins 90 % en nombres desdits globules huileux présentent une taille mesurée au microscope optique comprise entre 100 et 1000 nm, de préférence entre 100 et 500 nm.

7.  Emulsion selon l'une quelconque des revendications 4 à 6, caractérisée en ce que la teneur en agent(s) émulsionnant(s) est comprise entre 0,5 et 20 % en poids, de préférence entre 2 et 10 % en poids, par rapport au poids total de la composition.

8.  Emulsion selon l'une quelconque des revendications 4 à 7, caractérisée en ce qu'elle contient en outre au moins un adjuvant choisi parmi les épaississants ioniques ou non ioniques, les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants et les colorants.

9.  Emulsion selon l'une quelconque des revendications 4 à 8, caractérisée en ce que la teneur en poids de la phase aqueuse représente de 50 à 95 %, de préférence de 70 à 90 %, du poids total de la composition.

10. Emulsion selon l'une quelconque des revendications 4 à 9, caractérisée en ce que la teneur en poids de la phase huileuse représente de 5 à 50 %, de préférence de 10 à 30 %, du poids total de la composition.

11. Emulsion selon l'une quelconque des revendications 4 à 10, caractérisée en ce que la teneur en poids de la dihydroxyacétone représente de 0,5 à 10 %, de préférence de 1 à 7 %, du poids total de la composition.

12. Utilisation d'une émulsion ultrafine de type huile-dans-eau définie selon l'une quelconque des revendications 4 à 11 comme, ou pour la fabrication de, compositions cosmétiques destinées au bronzage et/ou au brunissage artificiels de la peau.

13. Composition cosmétique à usage topique utilisable pour le bronzage et/ou le brunissage artificiels de la peau, comprenant au moins de la dihydroxyacétone dans un support constitué d'une émulsion ultrafine de type huile-dans-eau définie selon l'une quelconque des revendications 4 à 11.

14. Procédé de traitement cosmétique pour bronzer et/ou brunir artificiellement la peau, caractérisé en ce qu'il consiste à appliquer sur celle-ci une quantité efficace d'une composition telle que définie dans la revendication 13.

**Patentansprüche**

1. Verfahren zur Herstellung einer ultrafeinen Emulsion vom Typ Öl-in-Wasser ohne Lipidvesikel, die mindestens Dihydroxyaceton enthält und in der die mittlere Größe der Kügelchen, die die Ölphase der Emulsion bilden, bestimmt mit einem optischen Mikroskop im Bereich von 100 bis 1000 nm liegt, dadurch gekennzeichnet, dass es die folgenden Schritte umfasst:

(i) In Gegenwart eines geeigneten emulgierenden Systems und unter Rühren werden eine Fettphase und eine wässerige Phase vermischt, wobei das Mischen bei einer Temperatur erfolgt, die über der Phasen-Inversionstemperatur (PIT) des Mediums liegt, um eine Emulsion vom Typ Wasser-in-Öl zu erhalten,
(ii) die Temperatur der so erhaltenen Emulsion wird auf eine Temperatur unter der Phasen-Inversionstemperatur gebracht, wodurch eine ultrafeine Emulsion vom Typ Öl-in-Wasser erhalten wird, und
(iii) bei der Durchführung des Schritts (i) und/oder nach Schritt (ii), jedoch vorzugsweise nach Schritt (ii), wird Dihydroxyaceton zugegeben.

2. Verfahren nach Anspruch 1, dadurch gekennzeichnet, dass der Emulgator oder die Emulgatoren vom nichtionischen Typ sind und unter polyethoxylierten und/oder polypropoxylierten Fettalkoholen und Estern von Fettsäuren und Polyolen, die gegebenenfalls polyethoxyliert und/oder polypropoxyliert sind, ausgewählt sind, die allein oder im Gemisch vorliegen.

3. Verfahren nach Anspruch 2, dadurch gekennzeichnet, dass das emulgierende System insgesamt einen HLB-Wert im Bereich von 9,5 bis 11,5 und vorzugsweise nahe 10 aufweist.

4. Ultrafeine Emulsion vom Typ Öl-in-Wasser ohne Lipidvesikel, die mindestens Dihydroxyaceton enthält und in der die mittlere Größe der Ölkügelchen, bestimmt mit einem optischen Mikroskop, im Bereich von 100 bis 1000 nm liegt und die nach dem Verfahren nach einem der Ansprüche 1 bis 3 hergestellt werden kann.

5. Emulsion nach Anspruch 4, dadurch gekennzeichnet, dass diese mittlere Größe im Bereich von 100 bis 500 nm liegt.

6. Emulsion nach einem der Ansprüche 4 oder 5, dadurch gekennzeichnet, dass mindestens 90 % der Anzahl der Ölkügelchen eine mit einem optischen Mikroskop bestimmte Größe von 100 bis 1000 nm und vorzugsweise von 100 bis 500 nm aufweisen.

7. Emulsion nach einem der Ansprüche 4 bis 6, dadurch gekennzeichnet, dass der Anteil des Emulgators oder der Emulgatoren im Bereich von 0,5 bis 20 Gew.-% und vorzugsweise von 2 bis 10 Gew.-%, bezogen auf das Gesamtgewicht der Zusammensetzung, liegt.

**8.** Emulsion nach einem der Ansprüche 4 bis 7, dadurch gekennzeichnet, dass sie ferner mindestens einen Zusatzstoff enthält, der ausgewählt ist unter ionischen oder nichtionischen Verdickungsmitteln, reizlindernden Mitteln, Antioxidantien, Trübungsmitteln, Stabilisatoren, Emollentien, insektenabwehrenden Mitteln, im UV-A-Bereich und/oder UV-B-Bereich wirksamen organischen Sonnenschutzfiltern, anorganischen Pigmenten und Nanopigmenten zum Lichtschutz, Hydratisierungsmitteln, Vitaminen, Parfums, Konservierungsmitteln, Füllstoffen, Maskierungsmitteln und Färbemitteln.

**9.** Emulsion nach einem der Ansprüche 4 bis 8, dadurch gekennzeichnet, dass der Gewichtsanteil der wässerigen Phase 50 bis 95 % und vorzugsweise 70 bis 90 % des Gesamtgewichts der Zusammensetzung ausmacht.

**10.** Emulsion nach einem der Ansprüche 4 bis 9, dadurch gekennzeichnet, dass der Gewichtsanteil der Ölphase 5 bis 50 % und vorzugsweise 10 bis 30 % des Gesamtgewichts der Zusammensetzung ausmacht.

**11.** Emulsion nach einem der Ansprüche 4 bis 10, dadurch gekennzeichnet, dass der Gewichtsanteil des Dihydroxyaceton 0,5 bis 10 % und vorzugsweise 1 bis 7 % des Gesamtgewichts der Zusammensetzung ausmacht.

**12.** Verwendung einer in einem der Ansprüche 4 bis 11 definierten ultrafeinen Emulsion vom Typ Öl-in-Wasser als kosmetische Zusammensetzung zum künstlichen Bräunen und/oder zur künstlichen Braunfärbung der Haut oder zu deren Herstellung.

**13.** Kosmetische Zusammensetzung zur topischen Anwendung, die zum künstlichen Bräunen und/oder zur künstlichen Braunfärbung der Haut verwendet werden kann und die in einem Träger, der aus einer in einem der Ansprüche 4 bis 11 definierten ultrafeinen Emulsion vom Typ Öl-in-Wasser besteht, mindestens Dihydroxyaceton enthält.

**14.** Verfahren zur kosmetischen Behandlung zum künstlichen Bräunen und/oder zur künstlichen Braunfärbung der Haut, dadurch gekennzeichnet, dass es darin besteht, auf die Haut eine wirksame Menge einer Zusammensetzung nach Anspruch 13 aufzutragen.

## Claims

**1.** Process for preparing an ultrafine emulsion of oil-in-water type free of lipid vesicles, which comprises at least dihydroxyacetone and of which the mean size of the globules which constitute the oily phase of the said emulsion, measured using an optical microscope, is between 100 nm and 1000 nm, characterized in that it comprises the following steps:

(i) a fatty phase, on the one hand, and an aqueous phase, on the other hand, are mixed together in the presence of a suitable emulsifying system and with stirring, the said mixing being carried out at a temperature above the phase inversion temperature (PIT) of the medium, so as to obtain an emulsion of water-in-oil type,
(ii) the temperature of the emulsion thus obtained is cooled to a temperature below the said phase inversion temperature, whereby an ultrafine emulsion of oil-in-water type is obtained,
(iii) dihydroxyacetone is introduced during step (i) and/or after step (ii), but preferably after step (ii).

**2.** Process according to Claim 1, characterized in that the emulsifier(s) is (are) of nonionic type and is (are) chosen, alone or as mixtures, from polyoxyethylenated and/or polyoxypropylenated fatty alcohols, and optionally polyoxyethylenated and/or polyoxypropylenated esters of fatty acid polyols

**3.** Process according to Claim 2, characterized in that the emulsifying system has an overall HLB of between 9.5 and 11.5 and preferably close to 10.

**4.** Ultrafine emulsion of oil-in-water type free of lipid vesicles, comprising at least dihydroxyacetone and of which the mean size of the oily globules, measured using an optical microscope, is between 100 nm and 1000 nm, which can be obtained according to the process defined in any one of Claims 1 to 3.

**5.** Emulsion according to Claim 4, characterized in that the said mean size is between 100 nm and 500 nm.

**6.** Emulsion according to either one of Claims 4 and 5, characterized in that at least 90%, in numerical terms, of the said oily globules have a size, measured using an optical microscope, of between 100 nm and 1000 nm and preferably between 100 nm and 500 nm.

7. Emulsion according to any one of Claims 4 to 6, characterized in that the content of emulsifier(s) is between 0.5% and 20% by weight and preferably between 2% and 10% by weight relative to the total weight of the composition.

8. Emulsion according to any one of Claims 4 to 7, characterized in that it also contains at least one adjuvant chosen from ionic or nonionic thickeners, softeners, antioxidants, opacifiers, stabilizers, emollients, insect repellents, organic sunscreens that are active in the UV-A and/or UV-B range, mineral sunblock pigments and nanopigments, moisturizers, vitamins, fragrances, preserving agents, fillers, sequestering agents and dyes.

9. Emulsion according to any one of Claims 4 to 8, characterized in that the weight content of the aqueous phase represents from 50% to 95% and preferably from 70% to 90% of the total weight of the composition.

10. Emulsion according to any one of Claims 4 to 9, characterized in that the weight content of the oily phase represents from 5% to 50% and preferably from 10% to 30% of the total weight of the composition.

11. Emulsion according to any one of Claims 4 to 10, characterized in that the weight content of dihydroxyacetone represents from 0.5% to 10% and preferably from 1% to 7% of the total weight of the composition.

12. Use of an ultrafine emulsion of oil-in-water type defined according to any one of Claims 4 to 11, as, or for the manufacture of, cosmetic compositions for artificially tanning and/or browning the skin.

13. Cosmetic composition for topical use which can be used for artificially tanning and/or browning the skin, comprising at least dihydroxyacetone in a support consisting of an ultrafine emulsion of oil-in-water type defined according to any one of Claims 4 to 11.

14. Cosmetic treatment process for artificially tanning and/or browning the skin, characterized in that it consists in applying an effective amount of a composition as defined in Claim 13 to the skin.